Europäisches Patentamt

European Patent Office

Office européen des brevets

(19)

(11) **EP 0 909 164 B1**

(12) **EUROPEAN PATENT SPECIFICATION**

(45) Date of publication and mention
of the grant of the patent:
**21.08.2002 Bulletin 2002/34**

(51) Int Cl.⁷: **A61K 9/127**

(86) International application number:
**PCT/EP97/02598**

(21) Application number: **97923963.9**

(22) Date of filing: **12.05.1997**

(87) International publication number:
**WO 97/042937 (20.11.1997 Gazette 1997/50)**

(54) **INSTANT VESICULAR PRODUCT**

**INSTANT VESIKULÄRES PRODUKT**

**PRODUIT VESICULAIRE INSTANTANE**

(84) Designated Contracting States:
**AT BE CH DE DK ES FI FR GB GR IE IT LI LU MC NL PT SE**
Designated Extension States:
**AL LT LV RO SI**

(30) Priority: **10.05.1996 EP 96201290**

(43) Date of publication of application:
**21.04.1999 Bulletin 1999/16**

(73) Proprietor: **YAMANOUCHI EUROPE B.V.**
**2350 AC Leiderdorp (NL)**

(72) Inventors:
• **MOLLEE, Hinderikus, Marius**
  **NL-2253 JL Voorschoten (NL)**
• **DE VRINGER, Tom**
  **NL-2713 RX Zoetermeer (NL)**

(56) References cited:
**EP-A- 0 521 562**         **EP-A- 0 678 295**
**WO-A-95/13795**          **WO-A-95/20945**
**DE-A- 4 021 083**

**Description**

The invention relates to an instant vesicular product, a process for the preparation thereof and compositions comprising the said product.

Vesicles in apolar vehicles were described in 1991 by H. Kunieda et al (J. Am. Chem. Soc. 113 (3) 1051-1052). The reversed vesicles, consisting essentially of the hydrophilic surfactant tetra-ethyleneglycol dodecyl ether in dodecane, were found to coalesce and revert back to a lamellar liquid crystalline phase over a period of hours to days, despite the addition of about 2.5 water molecules per ethyleneoxide-unit. Further publications on the same issue and by the same authors disclosed a preference for the use of straight chain hydrocarbon compounds as the apolar medium (H. Kunieda et al: Langmuir 1991 (7) 1915-1919, J. Coll. Interf. Sci 1991 (147) 286-288, J. Coll. Interf. Sci 1993 (156) 446-453). However, such dispersions have a very limited practical significance for product development in view of the very bad cosmetic and palatability properties of the said compounds, their toxic properties and also because these compounds are not biodegradable. International patent application WO 93/00069 disclosed dispersions of reversed vesicles in apolar vehicles, which vesicles were stable during a considerable period of time. The dispersions of vesicles disclosed therein were prepared by sonicating a mixture consisting of one or more surfactants, a lipophilic stabilising factor, optionally a hydrophilic stabilising factor and an apolar vehicle. The components for preparing the reversed vesicles in principle can be selected from a variety of materials. However, on substituting the hydrocarbon compounds by biodegradable apolar compounds, such as glycerol tri-esters of higher saturated and unsaturated fatty acids having 10-30 carbon atoms and vegetable oils, the yield of reversed vesicles, as assessed by polarised-light microscopy, is rather poor in the present inventors' experience, as compared to the yield when such vesicles are prepared in a hydrocarbon vehicle.

Since dispersions of reversed vesicles have shown distinct advantages over those of vesicles in aqueous vehicles, among other things a high encapsulating capacity for both lipophilic and hydrophilic drugs and a high encapsulating efficiency for hydrophilic drugs, there has been a demand to find a way to increase the yield of reversed vesicles in apolar pharmaceutically and cosmetically acceptable vehicles, such as the above-mentioned glycerol tri-esters and vegetable oils, without adversely affecting the encapsulating capacity and efficiency thereof. International patent application WO 95/20945 discloses a dispersion of reversed vesicles, which was prepared from specially processed galactolipids, obtained from oat kernels, in a MCT oil by sonication for 1 hour at 30-40°C. The presence of large reversed vesicles was assessed by means of a differential interference phase contrast microscope, but neither an assessment of the amount of vesicles formed was made nor a particle size distribution provided. The dispersions were said to be stable for about one week.

It has now been found that by making a primary dispersion of reversed vesicles in a suitable apolar vehicle and subsequently removing the said apolar vehicle a powder of reversed vesicles is obtained, which on dispersion in the same apolar vehicle maintains its vesicular structure and thus the dispersion of reversed vesicles is instantaneously obtained again. Surprisingly the same powder of reversed vesicles on dispersion in another apolar vehicle, such as a biodegradable oil, also maintains its vesicular structure and in this way a secondary dispersion of reversed vesicles is instantaneously obtained. The amount of reversed vesicles in the biodegradable oil appears to be very high as compared with the yield of reversed vesicles when these would have been prepared directly in the biodegradable oil.

The powder of reversed vesicles comprises one or more non-ionic surfactants and optionally a lipophilic stabilising factor, such as cholesterol. Other examples of compounds to be used as the lipophilic stabilising factor can be found in WO 93/00069. The product may further comprise a bio-active agent. The non-ionic surfactant is advantageously a derivative of a pentose, a hexose or an oligomer thereof, such as a fatty acid ester or a fatty alcohol ether. By preference the non-ionic surfactant is a fatty acid ester of a pentose, such as xylose, a hexose, such as glucose, fructose, galactose, mannose or maltitol, or an oligomer thereof, such as sucrose, lactose or lactulose. Since the pentoses and hexoses avail of more than one esterifiable hydroxy-group, the fatty acid esters of these compounds consist of a mixture of mono-, di-, tri- and poly-esters. Most preferably those products are used which contain at least 50 wt% of mono-esters and there is even more preference for those compounds, containing at least 70 wt% of mono-esters, the percentages based on the weight of the surfactant. The same applies to the corresponding ether compounds. Suitable fatty acids for the esterification are C8-C30 straight chain saturated and unsaturated fatty acids, such as lauric acid, myristic acid, palmitic acid, stearic acid, behenic acid and oleic acid. In particular and also in view of the commercial availability thereof fatty acid esters of sucrose are used for the preparation of the instant product. Suitable examples thereof are S-970 (sucrose stearate, 50% mono ester, 50% di-, tri- and poly ester); P-1570 (sucrose palmitate, 70% mono ester); S-1670 (sucrose stearate, 75% mono ester); M-1695 (sucrose myristate, 80% mono ester) and L-1695 (sucrose laurate, 80% mono ester).

The powder of reversed vesicles is prepared by a process, which comprises the steps of:

-    making a primary dispersion of reversed vesicles by sonication or microfluidisation of a mixture consisting of one or more non-ionic surfactants and optionally a lipophilic stabilising factor and a bio-active agent in a suitable apolar vehicle and

- subsequently removing the said apolar vehicle.

The reversed vesicles and/or the components, making up the vesicles, including the bio-active agent, are insoluble or practically insoluble in the apolar vehicle to be used for making the primary dispersion of reversed vesicles. The apolar vehicle is selected from compounds or mixtures thereof which, when evaporation techniques will be used, preferably have a high vapour pressure, in particular below the temperature at which the vesicles melt. Examples of such apolar vehicles are volatile silicone oils, such as Abil® K4, isoalkanes, such as isoparaffines, and (C1-C4)-alkyl alkanoates, such as ethyl acetate.

The primary dispersion of reversed vesicles may be prepared according to methods known in the art, e.g. such as disclosed in international patent application WO 93/00069. Preferably during the preparation of the primary dispersion of reversed vesicles a hydrophilic stabilising factor, such as water, is added. It appeared that small amounts thereof are sufficient to reduce the particle size of the vesicles and, as the result thereof, to increase the amount of reversed vesicles and the rate at which the reversed vesicles are formed. E.g. in case a sucrose ester is used as the non-ionic surfactant, an amount of up to 15 wt% of water, the percentage based on the weight of the surfactant, is advantageously used. More preferably 5-10 wt% of water is added during the preparation of the primary dispersion of reversed vesicles. As it appears to be the case, the water can be added at several stages during the preparation, but preferably it is present right at the beginning.

Removal of the apolar vehicle from the primary dispersion of reversed vesicles can be performed in several ways, such as by evaporation, centrifugation, filtration, lyophilisation etc. However, it is important that the bilayer structure of the vesicles will not be perturbed during the removal. There is a preference for evaporation techniques, in particular rotational evaporation and spray-drying. On using these processes it has appeared that the addition of excipients, such as the so-called cryoprotectants used during lyophilisation processes, is not necessary.

The product, obtained as described above and in details in the appended examples, consists of a vesicular structure, as a consequence of which a bio-active agent, if included in the primary dispersion of reversed vesicles, remains encapsulated. It may together with one or more excipients be incorporated in compositions, encompassing another aspect of the invention. The excipients may be solid in the form of dry powders or granulates in order to make tablets, capsules etc. The excipients may also be liquid or semi-solid in order to prepare dispersions. The liquid may be a polar compound, such as water or propylene glycol, or is a biodegradable compound. It has been demonstrated that in this way it is possible to make dispersions of reversed vesicles in a biodegradable apolar compound in a high amount, as compared to those directly made in the biodegradable compound according to the methods known in the art. Examples of such biodegradable natural or synthetic compounds are fatty acids, such as oleic acid, vegetable oils, such as peanut-oil and sesame oil, and mono-, di- and triglycerides of saturated and unsaturated, straight-chain fatty acids with 12 to 30 carbon atoms such as lauric acid, myristic acid, palmitic acid, stearic acid and arachidonic acid.

Whichever the way the product according to the present invention is incorporated into a composition, it is clear that the bad cosmetic and palatability properties of the non-volatile hydrocarbon apolar dispersion vehicle have been eliminated. Since the encapsulation efficiency of the reversed vesicles for bio-active agents is highly influenced by the choice of the apolar vehicle, it is a further advantage that the product according to the present invention is obtained using an apolar vehicle, which is a non-solvent, preferably also for the bio-active agent. On dispersion of the product in another apolar solvent to instantaneously obtain a secondary dispersion of reversed vesicles a high encapsulation efficiency of the bio-active agent has been found. Another advantage of the powder of reversed vesicles is the increase of stability of the various components and especially due to the structural integrity the prevention of leakage of bio-active compounds from the vesicles.

The following examples further illustrate the invention.

EXAMPLE 1

44.375 g of a silicone oil (Abil® K4), 5 g of sucrose palmitate P1570 (a mixture of 70% mono-esterified and 30% di- and poly-esterified sucrose, as obtained from C.N. Schmidt B.V., Amsterdam, The Netherlands), 0.5 g of cholesterol and 0.125 g p-aminobenzoic acid (PABA) were weighed into a thermostated vessel at 90°C. The mixture was sonified at 97 watts output during 30 minutes using a Branson sonifier 250 (Branson Ultrasonics Corp. Danbury, U.S.A.) followed by cooling of the vessel with cooling water of 7.5°C during 15 minutes until room temperature was reached. During cooling the mixture was sonified at 97 watts output with a duty cycle of 50%. Crystallisation of the silicone oil at the wall of the vessel was prevented by also stirring the mixture using a magnetic bar during cooling.

1.1 <u>Removal of apolar vehicle by rotational evaporation</u>

50 ml of the dispersion of vesicles so obtained was transferred to a 250 ml round bottom flask. The silicone oil was allowed to evaporate using a Büchi Rotavap (Büchi Laboratoriums AG, Flawil, Switzerland), the waterbath being kept at 30°C. The rotational speed of the round bottom flask was set at five and the pressure was reduced to 0.1 bar. After evaporation was completed, the remaining film was gathered and milled in a mortar.

1.2 Removal of apolar vehicle by spray drying

50 ml of the dispersion of vesicles was transferred to a mini spray dryer (Büchi 190, Büchi Laboratoriums AG Flawil, Switzerland), operating conditions: airflow 500 NL/h, inlet temperature 67°C, outlet temperature 56°C.

EXAMPLE 2

890 g of the silicone oil Abil® K4, 100 g of sucrose palmitate P1570 and 10 g of cholesterol were weighed in a thermostated vessel kept at 70°C. The components were mixed for ten minutes using an Ultra Turrax high shear mixer. After this pre-homogenisation the mixture was transferred to a M110 T Microfluidiser device operated at a pressure of 9000 PSI (Microfluidics Corp., Newton, U.S.A.) and several cycles were passed. The microfluidiser was thermostated at 30°C using a Neslab Exacal Ex-410 device (Neslab, Newington, U.S.A.). After the last cycle the dispersion was cooled using a Neslab Endocal RTE 220 flow-through cooling device (Neslab, Newington, U.S.A.), the temperature of the waterbath being set at 25°C.

2.1 Removal of apolar vehicle by rotational evaporation

50 ml of the dispersion of vesicles so obtained was transferred to a 250 ml round bottom flask. The silicone oil was allowed to evaporate using a Büchi Rotavap (Büchi Laboratoriums AG, Flawil, Switzerland), the waterbath being kept at 30°C. The rotational speed of the round bottom flask was set at five and the pressure was reduced to 0.1 bar. After evaporation was completed, the remaining film was gathered and milled in a mortar.

2.2 Removal of apolar vehicle by spray drying

50 ml of the dispersion of vesicles was transferred to a mini spray dryer (Büchi 190, Büchi Laboratoriums AG Flawil, Switzerland), operating conditions: airflow 500 NL/h, inlet temperature 67°C, outlet temperature 56°C.

EXAMPLE 3

1.125 g of the powdered product obtained according to example 1.1 and 8.875 g of an oil, selected from the group consisting of caprylic/capric triglyceride (Miglyol® 812N), peanut oil, castor oil, oleic acid and the volatile silicone oil Abil® K4, were weighed in a 20 ml sample vial. The mixture was stirred for 10 minutes at 150 rpm using a magnetic stirrer. The presence of reversed vesicles in the dispersions as so-called Maltese crosses was assessed by polarised light microscopy (Olympus® BH2 Tokyo Japan) immediately after preparation and after storage of the dispersions for 2 weeks at room temperature. The results have been listed in table 1.

Table 1

| powder dispersed in | appearance |
|---|---|
| Miglyol® 812N | reversed vesicles and a lot of agglomerates of reversed vesicles |
| Peanut-oil | reversed vesicles and a lot of agglomerates of reversed vesicles |
| Castor oil | reversed vesicles and a lot of agglomerates of reversed vesicles |
| Oleic acid | reversed vesicles and some agglomerates of reversed vesicles |
| Abil® K4 | reversed vesicles and a lot of agglomerates of reversed vesicles |

No change in the appearance was observed after storage during two weeks at room temperature.

EXAMPLE 4

1.125 g of the powdered product obtained according to example 1.1 and 8.875 g of an oil, selected from the group consisting of caprylic/capric triglyceride (Miglyol® 812N), peanut oil, castor oil and the volatile silicone oil Abil® K4, were weighed in a 20 ml sample vial. The mixture was stirred for 10 minutes at 150 rpm using a magnetic stirrer.

Directly after preparation the encapsulation efficiency EE of PABA, defined as the percentage PABA encapsulated per gram of reversed vesicles dispersion, was calculated by means of the formula:

$$EE = [1 - (f*FP/TP)]* 100\%$$

wherein:

f = the weight fraction of non-encapsulated apolar vehicle
FP = the concentration (mg/g) of PABA dissolved in the non-encapsulated apolar vehicle
TP = the concentration (mg/g) of PABA dissolved in the dispersion of reversed vesicles in the apolar vehicle.

The results have been listed in table 2.

Table 2

| Powder dispersed in: | Encapsulation efficiency (%) mean ± SD (n=3) |
|---|---|
| Miglyol® 812N | 71.8±14.2 |
| peanut oil | 71.2±0.9 |
| castor oil | 57.9±0.8 |
| Abil® K4 | 98.5±0.1 |

EXAMPLE 5

A dispersion of reversed vesicles in an apolar medium, which is caprylic/capric triglyceride (Miglyol® 812N), peanut oil, castor oil, oleic acid or the silicone oil Abil® K4 was made according to the method of example 1.
The appearance of the dispersions was assessed by means of a polarised-light microscope as described in example 3, immediately after preparation and after storage of the dispersions for two weeks at room temperature. The results have been listed in table 3.

Table 3

| dispersion of reversed vesicles directly prepared in | appearance |
|---|---|
| Miglyol® 812N | some giant reversed vesicles and a lot of non-vesicular material |
| Peanut-oil | some reversed vesicles, some PABA crystals and a lot of non-vesicular material |
| Castor oil | non-vesicular material only |
| Oleic acid | non-vesicular material only |
| Abil® K4 | reversed vesicles only |

No change in the appearance was observed after storage during two weeks at room temperature.
The encapsulation efficiency of PABA was not determined, due to the lack of (sufficient) vesicular material.

EXAMPLE 6

The silicone oil Abil® K4, sucrose palmitate P-1570, cholesterol and water in the amounts as indicated in table 4, were weighed into a thermostated reaction vessel of 100 ml at 88°C. The mixture was sonicated using a Branson Sonifier 250 equipped with a 4.8 mm diameter microtip at 88 Watts output for 30 minutes. Subsequently, the sample was cooled to ambient temperature using a cooling bath at 15°C. Cooling was performed for 20 minutes under stirring to prevent agglomeration.
Two batches were prepared, wherein the water was added 15 and 30 minutes respectively after sonication had started.
All batches were stirred in sealed glass vials at room temperature.
On dispersing the powder of reversed vesicles in Abil® K4 it was observed that the vesicular structure was not changed due to the removal of the apolar vehicle.

Table 4

| sucrose palmitate | 10 | 10 | 10 | 10 | 10 | 10 | 10 |
|---|---|---|---|---|---|---|---|
| cholesterol | 1 | 1 | 1 | 1 | 1 | 1 | 1 |
| silicone oil | 89 | 86,5 | 84 | 79 | 79 | 79 | 74 |
| water content (%) | 0 | 2,5 | 5 | 10 | 10 after 15 min | 10 after 30 min | 15 |
| particle size (microscopical) | 6-12 μm | 2-6 μm | 1-4 μm | 1-4 μm | 1-4 μm | 8-16 μm | 1-4 μm |
| particle amount | - | +/- | ++ | ++ | ++ | - | ++ |
| part.size distr. | - | - | +/- | + | +/- | + | + |
| waterdrops | - | - | - | - | - | + | + |
| crystals visible | - | - | - | - | - | - | - |
| sedimentation rate* | + | - | - | -- | - | ++ | -- |

* sedimentation rate: ++ very fast, -- slow

EXAMPLE 7

Dispersions of reversed vesicles, having the composition as shown in table 5, were prepared according to the method described in example 6. Instead of the reaction vessel a closed vessel, equipped with double walls, was used. The samples were cooled using a cooling bath at 7.5°C. In case ethyl acetate was used as the apolar vehicle the temperature at which the mixture was sonicated was reduced to 60°C. On dispersing the powder of reversed vesicles in Abil® K4, it was observed that the vesicular structure was not changed due to the removal of the apolar vehicle.

Table 5

| Reversed vesicle dispersions | | | | | | | | | |
|---|---|---|---|---|---|---|---|---|---|
| Components | | | | | | | | | |
| Sucrose palmitate P1570 | 10 | 10 | 10 | 10 | 10 | 10 | 10 | 10 | 10 |
| Cholesterol | - | 1 | 2 | - | 1 | 2 | - | 1 | 2 |
| Water | - | - | - | 1 | 1 | 1 | - | - | - |
| Isoparaffine (Isopar® E, obtained from Exxon Chemical International | 90 | 89 | 88 | 89 | 88 | 87 | - | - | - |
| Ethyl acetate | - | - | - | - | - | - | 90 | 89 | 88 |
| Mean particle size (nm) | 324 | 471 | 432 | 142 | 120 | 169 | 5138 | 1097 | 426 |
| Standard deviation | 10 | 15 | 58 | 12 | 8 | 3 | 6396 | 163 | 104 |

**EXAMPLE 8**

Dispersions of reversed vesicles, having the composition as shown in table 6, were prepared according to the method described in example 7.

After removal of the apolar vehicle 0.5 g of the powder of reversed vesicles was dispersed in 15 g of propylene glycol under stirring at 150 r.p.m. After 6 hours in all batches maltese crosses could be observed, which is comparable to the results obtained after dispersal of the same product in the silicone oil Abil® K4.

Table 6

| components | % | % | % | % |
|---|---|---|---|---|
| Sucrose palmitate | 10 | 10 | 10 | 10 |

Table 6   (continued)

| components | % | % | % | % |
|---|---|---|---|---|
| Cholesterol | 1 | 2 | 1 | 1 |
| Water | - | - | 0.5 | 1 |
| Abil® K4 | 89 | 88 | 88.5 | 88 |

**Claims**

1.  Powder of reversed vesicles, which comprises one or more non-ionic surfactants.

2.  Powder according to claim 1, **characterised in that** the non-ionic surfactant is a derivative of a pentose, a hexose or an oligomer thereof.

3.  Powder according to claim 2, **characterised in that** the derivative of the pentose, hexose or oligomer thereof is a fatty acid ester or a fatty alcohol ether.

4.  Powder according to claim 3, **characterised in that** the fatty acid ester of the pentose, hexose or oligomer thereof consists of a mono-ester for at least 50 wt%, the percentage based on the weight of the surfactant.

5.  Powder according to claim 4, **characterised in that** the mono-ester is present for at least 70 wt%, the percentage based on the weight of the surfactant.

6.  Powder according to any one of claims 1-5, **characterised in that** the non-ionic surfactant is a fatty acid ester of sucrose.

7.  Powder according to any one of claims 1-6, **characterised in that** it further contains a lipophilic stabilising factor.

8.  Powder according to any one of claims 1-7, **characterised in that** it encapsulates a bio-active compound.

9.  Process for the preparation of the powder according to any one of claims 1-8, which comprises making a dispersion of reversed vesicles from the non-ionic surfactant(s) and optionally the lipophilic stabilising factor and the bioactive agent in an apolar vehicle, **characterised in that** the apolar vehicle is subsequently removed.

10. Process according to claim 9, **characterised in that** the apolar vehicle is removed by evaporation techniques.

11. Process according to claim 9 or 10, **characterised in that** the apolar vehicle is a volatile compound.

12. Process according to any one of claims 9-11, **characterised in that** the volatile compound is selected from the group consisting of silicone oils, isoparaffins and (C1-C4)-alkyl alkanoates.

13. Process according to any one of claims 9-12, **characterised in that** a hydrophilic stabilising factor in an amount of up to 15 wt%, the percentage based on the weight of the surfactant, is added during the preparation of the dispersion of reversed vesicles.

14. Process according to claim 13, **characterised in that** the hydrophilic stabilising factor is added in an amount of between 5 and 10 wt%, the percentage based on the weight of the surfactant.

15. Process according to claim 13 or 14, **characterised in that** as the hydrophilic stabilising factor water is used.

16. Composition, prepared with the powder according to any one of claims 1-8 or with the product obtained by the process according to any one of claims 9-15.

17. Process for the preparation of a dispersion of reversed vesicles in a biodegradable oil, **characterised in that** the powder according to any one of claims 1-8 or the product obtained according to any one of claims 9-15 is dispersed in the biodegradable oil.

**Patentansprüche**

1. Pulver aus umgekehrten Vesikeln, umfassend ein oder mehrere nichtionische oberflächenaktive Mittel.

2. Pulver nach Anspruch 1, **dadurch gekennzeichnet, dass** das nichtionische oberflächenaktive Mittel ein Derivat einer Pentose, einer Hexose oder eines Oligomeren davon ist.

3. Pulver nach Anspruch 2, **dadurch gekennzeichnet, dass** das Derivat der Pentose, Hexose oder des Oligomeren davon ein Fettsäureester oder ein Fettalkoholether ist.

4. Pulver nach Anspruch 3, **dadurch gekennzeichnet, dass** der Fettsäureester der Pentose, der Hexose oder des Oligomeren davon aus einem Monoester zu mindestens 50 Gew.-%, bezogen auf das Gewicht des oberflächenaktiven Mittels, besteht.

5. Pulver nach Anspruch 4, **dadurch gekennzeichnet, dass** der Monoester in einer Menge von mindestens 70 Gew.-% vorhanden ist, wobei der Prozentgehalt auf das Gewicht des oberflächenaktiven Mittels bezogen ist.

6. Pulver nach einem der Ansprüche 1 bis 5, **dadurch gekennzeichnet, dass** das nichtionische oberflächenaktive Mittel ein Fettsäureester von Saccharose ist.

7. Pulver nach einem der Ansprüche 1 bis 6, **dadurch gekennzeichnet, dass** es weiterhin einen lipophilen Stabilisierungsfaktor enthält.

8. Pulver nach einem der Ansprüche 1 bis 7, **dadurch gekennzeichnet, dass** es eine bioaktive Verbindung einkapselt..

9. Verfahren zur Herstellung des Pulvers nach einem der Ansprüche 1 bis 8, umfassend die Herstellung einer Dispersion von umgekehrten Vesikeln aus dem bzw. den nichtionischen oberflächenaktiven Mittel(n) und gegebenenfalls dem lipophilen Stabilisierungsfaktor und des bioaktiven Mittels in einem apolaren Träger, **dadurch gekennzeichnet, dass** der apolare Träger anschließend entfernt wird.

10. Verfahren nach Anspruch 9, **dadurch gekennzeichnet, dass** der apolare Träger durch Abdampftechniken entfernt wird.

11. Verfahren nach Anspruch 9 oder 10, **dadurch gekennzeichnet, dass** der apolare Träger eine flüchtige Verbindung ist.

12. Verfahren nach einem der Ansprüche 9 bis 11, **dadurch gekennzeichnet, dass** die flüchtige Verbindung aus der Gruppe bestehend aus Silikonölen, Isoparaffinen und (C1-C4)-Alkylalkanoaten ausgewählt wird.

13. Verfahren nach einem der Ansprüche 9 bis 12, **dadurch gekennzeichnet, dass** ein hydrophiler Stabilisierungsfaktor in einer Menge von bis zu 15 Gew.-%, wobei sich die Prozentangabe auf das Gewicht des oberflächenaktiven Mittels bezieht, während der Herstellung der Dispersion der umgekehrten Vesikel zugesetzt wird.

14. Verfahren nach Anspruch 13, **dadurch gekennzeichnet, dass** das hydrophile Stabilisierungsmittel in einer Menge von zwischen 5 und 10 Gew.-%, wobei die Prozentangabe sich auf das Gewicht des oberflächenaktiven Mittels bezieht, zugesetzt wird.

15. Verfahren nach Anspruch 13 oder 14, **dadurch gekennzeichnet, dass** als hydrophiler Stabilisierungsfaktor Wasser verwendet wird.

16. Zusammensetzung, hergestellt mit dem Pulver nach einem der Ansprüche 1 bis 8 oder mit dem Produkt, das durch das Verfahren nach einem der Ansprüche 9 bis 15 erhalten worden ist.

17. Verfahren zur Herstellung einer Dispersion von umgekehrten Vesikeln in einem bioabbaubaren Öl, **dadurch gekennzeichnet, dass** das Pulver nach einem der Ansprüche 1 bis 8 oder das Produkt, das nach einem der Ansprüche 9 bis 15 erhalten worden ist, in dem bioabbaubaren Öl dispergiert wird.

**Revendications**

1. Poudre constituée de vésicules inversées, qui contient un ou plusieurs agents tensioactifs non-ioniques.

2. Poudre selon la revendication 1, **caractérisée en ce que** l'agent tensioactif non-ionique est un dérivé d'un pentose, d'un hexose ou d'un oligomère de ceux-ci.

3. Poudre selon la revendication 2, **caractérisée en ce que** le dérivé du pentose, de l'hexose, ou d'un oligomère de ceux-ci est un ester d'acide gras ou un éther d'alcool gras.

4. Poudre selon la revendication 3, **caractérisée en ce que** l'ester d'acide gras du pentose, de l'hexos ou de l'oligomère de ceux-ci est constitué d'un mono-ester pour au moins 50% en poids, le pourcentage étant basé sur le poids de l'agent tensioactif.

5. Poudre selon la revendication 4, **caractérisée en ce que** le mono-ester est présent pour au moins 70% en poids, le pourcentage étant basé sur le poids de l'agent tensioactif.

6. Poudre selon une quelconque des revendications 1 à 5, **caractérisée en ce que** l'agent tensioactif non-ionique est un ester d'acide gras de sucrose.

7. Poudre selon une quelconque des revendications 1 à 6, **caractérisée en ce qu'**elle contient en outre un facteur lipophile de stabilisation.

8. Poudre selon une quelconque des revendications 1 à 7, **caractérisée en ce qu'**elle renferme un composé bio-actif.

9. Procédé de préparation de la poudre selon une quelconque des revendications 1 à 8, qui comprend la réalisation d'une dispersion de vésicules inversées à partir de l'agent (des agents) tensioactif(s) non-ionique(s) et facultativement à partir du facteur lipophile de stabilisation et à partir de l'agent bio-actif dans un véhicule apolaire, **caractérisé en ce que** le véhicule apolaire est consécutivement éliminé.

10. Procédé selon la revendication 9, **caractérisé en ce que** le véhicule apolaire est éliminé par des techniques d'évaporation.

11. Procédé selon la revendication 9 ou 10, **caractérisé en ce que** le véhicule apolaire est un composé volatil.

12. Procédé selon une quelconque des revendications 9 à 11, **caractérisé en ce que** le composé volatil est sélectionné parmi le groupe constitué d'huiles de silicone, d'isoparaffines et d'alcanoates d'alkyle en $C_1$-$C_4$.

13. Procédé selon une quelconque des revendications 9 à 12, **caractérisé en ce qu'**un facteur hydrophile de stabilisation en une teneur allant jusqu'à 15% en poids, le pourcentage étant basé sur le poids de l'agent tensioactif, est ajouté pendant la préparation de la dispersion de vésicules inversées.

14. Procédé selon la revendication 13, **caractérisé en ce que** le facteur hydrophile de stabilisation est ajouté en une teneur comprise entre 5 et 10% en poids, le pourcentage étant basé sur le poids de l'agent tensioactif.

15. Procédé selon la revendication 13 ou 14, **caractérisé en ce qu'**on utilise de l'eau comme facteur hydrophile de stabilisation.

16. Composition préparée avec la poudre selon une quelconque des revendications 1 à 8 ou avec le produit obtenu par le procédé selon une quelconque des revendications 9 à 15.

17. Procédé pour la préparation d'une dispersion de vésicules inversées dans une huile biodégradable, **caractérisé en ce que** la poudre selon une quelconque des revendications 1 à 8 ou le produit obtenu selon une quelconque des revendications 9 à 15 est dispersé(e) dans l'huile biodégradable.